# EUROPEAN PATENT SPECIFICATION

(11) **EP 3 983 477 B1**
(45) Date of publication and mention of the grant of the patent: **02.11.2022**
(21) Application number: 20730169.8
(22) Date of filing: 11.06.2020
(51) Int. Cl.: C08K 3/105, C08K 3/16, C08K 3/32, C08K 5/098, C08L 89/06, C08J 5/18, A61K 9/48, A61K 47/02, A61K 47/36, A61K 47/42, C09D 11/03, C09D 11/08, C09D 11/14, C08L 5/00, C08K 3/30, C08K 3/28

(54) **FILM, CAPSULE AND FILM-FORMING COMPOSITION COMPRISING WATER-SOLUBLE SALT AS OPACIFYING AGENT**
FILM, KAPSEL UND FILMBILDENDE ZUSAMMENSETZUNG MIT WASSERLÖSLICHEM SALZ ALS TRÜBUNGSMITTEL
FILM, CAPSULE ET COMPOSITION DE FORMATION DE FILM COMPRENANT UN SEL SOLUBLE DANS L'EAU UTILISÉ EN TANT QU'AGENT OPACIFIANT

(30) Priority: 12.06.2019 JP 2019109231; 04.09.2019 EP 19195336
(43) Date of publication of application: 20.04.2022
(73) Proprietor: Capsugel Belgium NV, 2880 Bornem (BE)
(72) Inventor: TAKUBO, Takahisa, Ayase-Shi, Kanagawa prefecture 252--1113 (JP); DOI, Hitomi, Kawasaki-Shi, Kanagawa prefecture 214--0003 (JP); SATO, Kaori, Kawasaki-shi, Kanagawa prefecture 215--0022 (JP)
(74) Representative: De Clercq & Partners
(86) International application number: PCT/EP2020/066201
(87) International publication number: WO 2020/249672

(56) References cited:
- WO-A1-2013/006470
- US-A1- 2009 010 975
- US-A1- 2014 135 352
- DATABASE WPI Week 201879 Thomson Scientific, London, GB; AN 2018-79865R XP002797856, & CN 108 576 785 A (UNIV YANGZHOU) 28 September 2018 (2018-09-28)

## Description

### Technical Field

The present invention relates to a film-forming composition for use in pharmaceutical preparations and the like which turns white by a salt in forming a film. The present invention also relates to capsules, oral dispersible films produced using the film-forming composition, preparations such as tablets and granules coated with the film-forming composition and applications such as film products and print inks.

The present invention further relates to light-shielding capsules produced from a film-forming composition providing a high degree of whiteness or opacity, capable of shielding light and ultraviolet rays, and maintaining stability and quality of an active ingredient for a long term.

### Background Art

Conventionally, preparations such as medicines and healthy foods have been colored such that the preparations are made highly distinguishable, have good appearance and maintain quality by shielding light. Particularly, preparations containing an unstable component to light are usually coated with a light-shielding white film, which is prepared by adding a light-shielding agent to a capsule film and a coating film. Further, if necessary, a light-shielding film is stained with a desired color by adding an edible dye (e.g., blue No. 1, yellow No. 5, red No. 3) or a pigment such as iron sesquioxide or aluminum lake.

Various types of opacifying agents are used for shielding light. The opacifying agent most frequently used is titanium dioxide. Titanium dioxide is a white pigment characterized in excellent whiteness, shielding power, coloring power and extremely high chemical stability and has long been used in the fields of medicine and cosmetic.

However, some of the components blended in these medicines and cosmetics lose stability by titanium dioxide and some are facilitated to decompose by radicals generated from titanium dioxide by application of UV rays. Likewise, there is a problem in quality maintenance. Also, in preparations covered with titanium dioxide-containing film, the film peels and degradation appears by irradiation with light over time, with the result that it becomes difficult to keep a coating state and appearance is destroyed. Other than these, components in preparations are decomposed by light, oxygen and a moisture content. This is another concern.

Then, as a coating material for pharmaceutical preparations, a coating material containing titanium dioxide and a radical scavenger (Patent Document 1), a titanium dioxide-free coating material, i.e., opaque or translucent hard capsules containing natural calcium powders having an average particle size of 3 µm or less in gelatin (Patent Document 2) and a coating agent containing talc and/or barium sulfate (Patent Document 3) have been investigated.

However, the inorganic pigments such as titanium dioxide are all insoluble in water and organic solvents, and thus, it is difficult to prepare coating liquids. In addition, since a suspension solution is sprayed, tablet surfaces become rough and the strength of a coating film decreases; a spray nozzle clogs during a production process; and solid matter is accumulated on a nozzle tip, causing liquid dripping. Causing these troubles in a coating step is a problem.

In consideration of this problem, a composition for a light-shielding film not containing titanium dioxide but containing at least one selected from water-soluble calcium salts, and a water-soluble cellulose polymer as a film base, has been studied (Patent Document 4).

However, the amount of the water-soluble calcium salt described in Patent Document 4 is limited to be 1.0% or less (in terms of calcium) of the food by the "Standards and criteria for food and food additives, etc., No.2 additive". Because of this, a problem was that the white coloring concentration is limited.

Further, a water-soluble salt such as sodium dihydrogen phosphate (Patent Document 5) or a water-soluble metal compound containing a monovalent, divalent, or trivalent metal (Patent Document 6) have been investigated as a light-shielding or an opacifying agent. However, these inventions described in Patent Documents 5 and 6 are directed to opacification of a cellulose derivative base film such as hydroxypropylmethylcellulose, and they are silent about opacification of gelatin or pullulan film.

Regarding gelatin base film, Patent document 7 describes that a water-soluble or water-insoluble carbonate or bicarbonate is added to the film to give a rapid release property. The carbonate in the film dissolves in the medium when exposed to an aqueous acidic environment. However, Patent document 7 does not teach or suggest to use a water-soluble salt as an opacifying agent for a gelatin film.

### Citation List

### Patent Documents

[Patent Document 1] Japanese Patent Laid-Open No. 11-147819
[Patent Document 2] Japanese Patent Laid-Open No. H7-124462
[Patent Document 3] Japanese Patent Laid-Open No. 2002-212104
[Patent Document 4] WO2004/054619A1
[Patent Document 5] CN 108 576 785 A
[Patent Document 6] US 2009/010975A1
[Patent Document 7] WO 2013/006470A1

### Summary of Invention

### Problems to be Solved by the Invention

Techniques using an alternative to titanium dioxide have been developed as described above; however, titanium dioxide particles are very fine, and their high shielding ability is incomparable. Since titanium dioxide produces a sufficient shielding ability in a small amount, a titanium dioxide powder has currently and widely been used as a light-shielding agent in many industrial fields including foods and medicines.

The International Agency for Research Cancer (IARC) previously classified carcinogenicity of titanium dioxide in group 3 (not classifiable as to its carcinogenicity to humans); however, the agency concluded in June, 2006, that the results of animal tests performed by introducing pigment-grade and ultrafine TiO₂ by inhalation and intratracheal injection are determined to sufficiently demonstrate carcinogenicity of TiO₂ in animals, and classified TiO₂ in group 2B (possibly carcinogenic to humans) (Baan R, Staif K, Grosse Y, Secretan B, Ghissassi F, Cogliano V. on behalf of the WHO International Agency for Research on Cancer Monograph Working Group (2006) Carcinogenicity of carbon black, titanium dioxide, and talk. [cited June 22, 2009], available from; http://oncology.thelancet.com.)

Because of this, particularly in the health food industry, use of capsules containing titanium dioxide is gradually avoided and development of an alternative light-shielding agent for titanium dioxide has been more and more desired. However, as mentioned above, an alternative to titanium dioxide excellent as a light-shielding agent cannot be easily found. Light-shielding capsules using no titanium dioxide, which are particularly desired in the health food industry, have not yet been supplied at present.

### [Means for Solving the Problems]

The present inventors have dealt with the aforementioned technical problems in order to provide an alternative light-shielding means replaceable for titanium dioxide particularly desired by the health food industry. As a result of screening and experiments for several years, they found that the film turns white in a film formation process by using colorless salts according to the type of a film-forming polymer in combination, and that good appearance comparable to the film having titanium dioxide dispersed therein and light-blocking property can be realized. They further found that the color does not change in a high-temperature and high-humidity environment. In the opacifying technology of the invention, the degree of whiteness or degree of light-shielding can be controlled by varying the amount of a salt to be added. Other than this, a desired color can be produced if a coloring material is used in combination.

Salts (e.g., malate, succinate, citrate, phosphate, carbonate, acetate) are widely used in foods as, e.g., a seasoning, a pH regulator, an acidulant, an antioxidant, a thickener, a stabilizer, a chelating agent, a dispersant and a buffer, and used in pharmaceuticals as, e.g., a taste-masking agent, a stabilizer, a buffer, a binder, a suspension agent, a tonicity agent, a pH regulator, a foaming agent, a disintegrant, a disintegration aid, a soothing agent, a solubilizer and a solubilization agent.

These are mostly colorless and transparent components. It is known that a film formed of a film-forming polymer turns white by the presence of a salt such as a water-insoluble barium sulfate and a water-soluble calcium salt (see, Patent Documents 3 and 4). However, the above technics are just directed to cellulose derivative base film. The inventors of the application has found that gelatin and pullulan films can be whitened or opacified by a water-soluble salt. This is a phenomenon surprisingly found, for the first time, by the present inventors.

Barium sulfate according to the conventional technology is significantly inferior to titanium dioxide in the degree of the resultant whiteness, because a barium sulfate powder insoluble in water is just dispersed to reduce a light-transmittance in place of titanium dioxide. If a calcium salt is used, it takes a long time to dry the resultant film and the amount of a calcium salt as a food additive is limited. However, the salt(s) of the present invention are free from such problems. Owing to the present invention, it is possible to provide beautiful white or light-shielding color films and capsules without using titanium dioxide. In particular, for the health food market, if a natural colorant derived from e.g., gardenia is used in combination, safe, secure and beautiful colored light-shielding capsules can be provided.

The technology of the invention can be applied to compositions containing a raw material ordinarily used for a capsule film, such as gelatin and pullulan. Other than the capsule film, these are also applicable, for the purpose of coloring, to band seal of capsules, intraoral dispersible film preparations, coating for tablets and grains, edible film products and food inks. The technology of the invention may be applied to a film formed by adding a salt to a solution of a polymer capable of forming a film, followed by drying, other than the aforementioned ones.

The degree of whiteness or opacity can be controlled by the type and amount of salt to be used, and the salt to be used in combination. Thus, the degree of whiteness or opacity can be changed from white having an excellent light-shielding property to dull white.

Mechanism of opacifying a film is unknown. However, when the film is formed by using a predetermined salt in combination with a polymer and observed by a microscope, it was observed that microcrystals are produced in a film base during a drying process and diffuse over the whole film. From the observation, opacifying presumably occurs due to continuous production of microcrystals of the salt in a film base in a drying process.

The base component for a polymer which can be used in the state of an aqueous solution in the present invention may be those available as a capsule base for pharmaceutical products and foods, and those available in coating for medicines and food preparations. Examples of the polymer include gelatin and pullulan.

Examples of the salt to be used in the present invention include a sodium salt, a potassium salt, an ammonium salt or a magnesium salt. Examples of the sodium salt include sodium malate, sodium citrate, disodium hydrogen phosphate, sodium dihydrogen phosphate, sodium succinate, sodium polyphosphate, sodium pyrophosphate. Examples of the potassium salt include dipotassium hydrogen phosphate, potassium dihydrogen phosphate and potassium acetate . Examples of the ammonium salt include ammonium chloride. Examples of the magnesium salt include magnesium chloride and magnesium sulfate.

These salts can be used singly or in combinations of two or more thereof. The degree of whiteness or opacity can be varied depending on the amount to be added.

Usually, the degree of whiteness or opacity can be changed with a total amount of the salt being in the range of 0.5 to 30 parts by weight with respect to a total amount of 100 parts by weight of the film-forming polymer component in terms of dry weight (for example, a polymer component(s) selected from gelatin and pullulan); acceptable within the range of 1 to 30 parts by weight, 2 to 30 parts by weight or 3 to 30 parts by weight; and preferably within the range of 1 to 15 parts by weight, 2 to 15 parts by weight or 3 to 15 parts by weight. If the range is beyond 30 parts by weight, the film tends to be brittle and easily broken. In contrast, if the range is less than 0.5 parts by weight, the film is not whitened or thin white, with the result that light-shielding cannot be expected.

To the composition of the present invention for forming a film including hard capsules, additives such as a gelling agent, a gelling aid, a plasticizer, a pH regulator, a sweetener, an acidulant, a preservative, a fragrance, a binder, a thickener and colorants except white can be appropriately added depending on the type of film-forming polymer component and intended use thereof, including. The amount of the gelling agent to be used falls within the range of 0 to 10% and preferably 0 to 5%.

As the pigments except white, for example, a black pigment and chromatic pigments may be blended. The chromatic color herein refers to all colors except black, white and gray.

If medicines or health food preparations are coated, any chromatic pigments may be acceptable as long as they can be used in the fields of medicine or food. Examples of the pigments include a lake tar pigment, a water-soluble tar pigment and a natural colorant.

Examples of the lake tar pigment include edible blue No. 1 aluminum lake, edible blue No. 2 aluminum lake, edible red No. 2 aluminum lake, edible red No. 3 aluminium lake, edible red No. 40 aluminum lake, edible yellow No. 4 aluminum lake, edible yellow No. 5 aluminum lake and edible green No. 3 aluminum lake. Examples of the water-soluble tar pigment include edible blue No. 1, edible blue No. 2, edible yellow No. 4, edible yellow No. 5, edible red No. 2, edible red No. 3, edible red No. 40, edible red No. 102, edible red No. 104, edible red No. 105, edible red 106 and edible green No. 3. Examples of the natural colorant include caramel colorant, gardenia colorant, safflower colorant, turmeric colorant, Benikouji colorant, carotene, beet-red, tomato colorant, cochineal colorant, lac colorant, perilla colorant, red cabbage colorant, red radish colorant, red rice colorant, azuki colorant, purple potato colorant, purple sticky potato colorant, purple corn colorant, grape skin colorant, various berry colorants, grape juice colorant, *haematococcus* alga colorant, red pepper colorant, annatto colorant, spirulina colorant, cocoa colorant, persimmon colorant, kouliang colorant, onion colorant, tamarind colorant, green tea powder, squid ink, *phaffia* colorant, pecan nut colorant, rutin, logwood colorant, saffron colorant, *Lonicera gracilipes glabra* colorant, tea colorants, nori colorant, hibiscus colorant, paprika powder, plum colorant, cherry colorant, chicory colorant, fruit juice, vegetable juice, red currant colorant, marigold colorant, chlorella powder, sodium norbixin, sodium iron chlorophyllin, sodium copper chlorophyllin, copper chlorophyll, a naked barley green leaf extract powder, naked barley green leaf juice dry powder and naked barley green leaf extract. Examples of other chromatic pigments include methylrosanilinium chloride, carmine, photosensitive element No. 201, permanent violet-R-special, methylene blue, riboflavin butyrate, riboflavin, riboflavin sodium phosphate, iron sesquioxide, yellow ferric oxide and black iron oxide. In order to provide a pearl luster, an appropriate amount of, e.g., a pearl pigment, which is produced from, e.g., a fine powder of fish scale foil, the nacreous layer of shellfish or mica, and silicon dioxide, can be used.

Examples of the gelling agent for use in the film-forming composition of the present invention include, but are not particularly limited to, carrageenan, gellan gum, agar, pectin, gelatin, xanthan gum, locust bean gum, curdlan, alginic acid, sodium alginate, guar gum, gum Arabic, glucomannan, tamarind seed gum, furcellaran, tara gum and karaya gum.

Examples of the plasticizer for use in the film-forming composition of the present invention include triethyl citrate, glycerin, D sorbitol (D-sorbitol/sorbitan solution), D-mannitol, trehalose, a vegetable oil (sesame oil, castor oil), a medium chain triglyceride, a triacetin, a phthalate (dioctyl phthalate), a phytosterol, a propylene glycol, polysorbate and a polyethylene glycol (macro goal).

Examples of the pH regulator include phosphoric acid, hydrochloric acid, citric acid, glycine, gluconic acid, succinic acid, acetic acid, tartaric acid, lactic acid, fumaric acid, boric acid, maleic acid, sulfuric acid, malic acid, ammonia, a hydroxide, an amine, and salts thereof.

Examples of the sweetener include aspartame, acesulfame potassium, amacha powder, liquid sugar, fructose, glucose, reduced maltitol syrup, licorice, xylitol, glycine, glycerin, glycyrrhizinate, brown sugar, saccharin, sucralose, stevia extract, refined white sugar, purification honey, D-sorbitol, maltitol, maltose and D-mannitol.

Examples of the acidulant include adipic acid, itaconic acid, citric acid, trisodium citrate, glucono-delta-lactone, gluconic acid, potassium gluconate, sodium gluconate, succinic acid, monosodium succinate, disodium succinate, sodium acetate, tartaric acid, lactic acid, sodium lactate, acetic acid, phytic acid, fumaric acid, malic acid and phosphoric acid. Examples of the preservative include benzoic acid, sodium benzoate, p-hydroxybenzoate, sodium sulfite, sodium hyposulfite, sodium pyrosulfite, potassium pyrosulfite, propionic acid, calcium propionate, sodium propionate, storax extract, capillary artemisia extract, Milt protein extract, a sorbic acid compound, sodium dehydroacetate, nysin, sulfur dioxide, a pectin degradation product and ε-polylysine.

Examples of the flavor include various essences, flavors, peppermint, menthol, peppermint, cinnamon, fennel and camphor.

The sweetener, acidulant and flavor are suitably used when a film is used for edible products.

Examples of the thickener include alginic acid, alginate, gum Arabic, karaya gum, guar gum, gellan gum, tamarind seed gum, tara gum, tragacanth gum, carrageenan, CMC-Ca, CMC-Na, glucosamine, pullulan, pectin, sodium polyacrylate, methylcellulose, curdlan and modified starch. The strength of a film can be increased by use of the thickener and binder.

The hard capsules according to the present invention can be produced by an ordinary method for producing capsules. A case of gelatin capsules will be described as an example. First, 20 to 40 parts by weight of gelatin is uniformly dissolved in hot water to obtain a light yellow (derived from gelatin) and transparent dipping fluid. The salt(s) of the present invention serving as an opacifying agent is added in this step to the dipping solution. After the viscosity of the fluid is adjusted, a molding pin made of stainless steel is soaked in the dipping solution and pulled up. The dipping solution on the pin is immediately cooled to form a gel. After an appropriate thickness suitable for a film for capsules is ensured, the film is dried, and unnecessary portion is cut away. A pair of the body and the cap are engaged to make a capsule.

Examples of the particularly preferable combination of the film base and the salt include gelatin and sodium polyphosphate, gelatin and sodium pyrophosphate, gelatin and sodium citrate, gelatin and disodium hydrogen phosphate, gelatin and sodium dihydrogen phosphate, gelatin and dipotassium hydrogen phosphate, gelatin and potassium dihydrogen phosphate, gelatin, sodium polyphosphate and sodium pyrophosphate, gelatin, sodium polyphosphate and disodium hydrogen phosphate.

A film is formed by dissolving a film base in hot water or cold water suitable for dissolution, adding a salt and further appropriately adding necessary additives depending on the purpose to obtain an aqueous solution, followed by heat or natural drying. The degree of whiteness or opacity may be affected by the temperature condition in a dry process. Accordingly, it is desirable to select the drying condition depending on the combination of the base and a salt.

Examples of the drying conditions include high temperature drying in an oven of 60°C for 15 minutes, high temperature drying in an oven of 60°C for 30 minutes or natural drying at room temperature.

### [Advantageous Effects of Invention]

According to the present invention, a film can be whitened or opacified by using a salt which has been conventionally used in the fields of medicine and food and not harmful in health but without using titanium dioxide, whose carcinogenicity is concerned. The degree of whiteness or opacity provided by the salt can be controlled from dull whiteness to the whiteness which gives the same light shielding property as titanium dioxide. Thus, the salt can be suitably used for an ordinary color forming process and for preparations of medicines and foods unstable to light.

The film produced from the film-forming composition of the present invention is excellent in solubility and can maintain stable whiteness or opacity for a long term. If a pigment is used together, an opaque colored film can be obtained.

Accordingly, particularly for the health food market, for example, if a colorant derived from a natural product represented by e.g., gardenia is used in combination, a safe, secure and beautifully colored light-shielding capsules and film can be provided.

### Brief Description of the Drawings

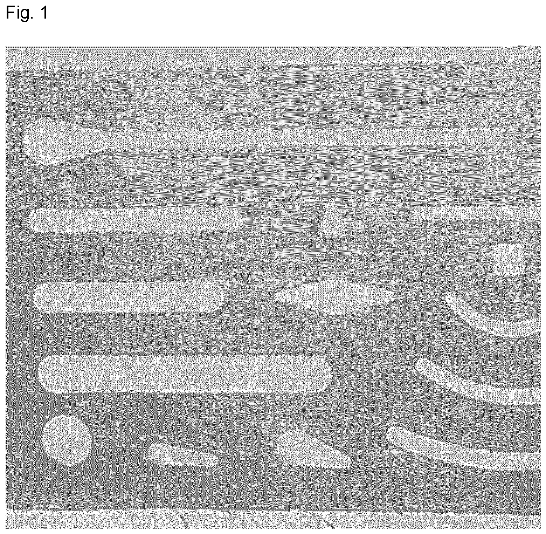

[Figure 1] Figure 1 shows a printed matter obtained by preparing a film-forming aqueous solution, which is obtained by adding, to an about 34% gelatin solution, 7.5 parts by weight of sodium polyphosphate relative to 100 parts by weight of the gelatin, printing patterns on a glass plate heated in an oven at 105°C by use of a template having pattern cuts and heating the plate at 60°C for 15 minutes.

### Experimental Example

Various types of test samples each containing a film-forming polymer and a salt were prepared and opacity of the films in a film-formation process was examined.

In the following tables, the contents of salts are represented by parts by weight relative to 100 parts by weight (on a dry basis) of a film base.

The white ratio and the degree of whiteness were visually evaluated based on the following criteria.

The "white ratio" represents the area percentage of whitened parts regardless of the "degree of whiteness"; whereas, the "degree of whiteness" represents the (color) density of a white-looking portion.

White ratio: ⊚ 100 to 90%, ∘90 to 80%, △ 80 to 70%, □ 70% or less

Degree of whiteness: ⊚ pure white, ∘ white, △ transparent-like white, □ dull white

Films were prepared by drying naturally in a room.

**Table 1**

| Examples of opacifying agents used with gelatin | | | | | | | | | |
|---|---|---|---|---|---|---|---|---|---|
| Formulation | 1 | 2 | 3 | 4 | 5 | 6 | 7 | 8 | 9 |
| Gelatin | 100 | 100 | 100 | 100 | 100 | 100 | 100 | 100 | 100 |
| Sodium polyphosphate | 20 | 10 | 5 | 3.5 | 2 | | | | |
| Sodium pyrophosphate | | | | | | 10 | 5 | | |
| Potassium carbonate | | | | | | | | 5 | |
| Sodium carbonate | | | | | | | | | 5 |
| Sodium bicarbonate | | | | | | | | | |
| Trisodium citrate | | | | | | | | | |
| Disodium hydrogen phosphate | | | | | | | | | |
| Sodium dihydrogen phosphate | | | | | | | | | |
| Dipotassium hydrogen phosphate | | | | | | | | | |
| Potassium dihydrogen phosphate | | | | | | | | | |
| White ratio | ⊚ | ⊚ | ⊚ | ⊚ | □ | ⊚ | ⊚ | □ | ⊚ |
| Degree of whiteness | ○ | △ | △ | △ | □ | △ | △ | □ | △ |

**Table 1 (continued)**

| Examples of opacifying agents used with gelatin | | | | | | | | | |
|---|---|---|---|---|---|---|---|---|---|
| Formulation | 10 | 11 | 12 | 13 | 14 | 15 | 16 | 17 | 18 |
| Gelatin | 100 | 100 | 100 | 100 | 100 | 100 | 100 | 100 | 100 |
| Sodium polyphosphate | | | | | | | | 2.5 | 2.5 |
| Sodium pyrophosphate | | | | | | | | 2.5 | |
| Potassium carbonate | | | | | | | | | |
| Sodium carbonate | | | | | | | | | |
| Sodium bicarbonate | 5 | | | | | | | | |
| Trisodium citrate | | 5 | | | | | | | |
| Disodium hydrogen phosphate | | | 10 | 5 | | | | | 2.5 |
| Sodium dihydrogen phosphate | | | | | 10 | | | | |
| Dipotassium hydrogen phosphate | | | | | | 10 | | | |
| Potassium dihydrogen phosphate | | | | | | | 10 | | |
| White ratio | □ | □ | ⊚ | ⊚ | ⊚ | ⊚ | ⊚ | ⊚ | ⊚ |
| Degree of whiteness | □ | △ | ○ | △ | □ | △ | □ | △ | △ |

Experiments were carried out using formulations 1 to 16 (shown in Table 1) consisting of a film base of gelatin and each of the salts: sodium polyphosphate, sodium pyrophosphate, potassium carbonate, sodium carbonate, sodium bicarbonate, trisodium citrate, disodium hydrogen phosphate, sodium dihydrogen phosphate, dipotassium hydrogen phosphate and potassium dihydrogen phosphate. Formulations 8 to 10 using a carbonate or bicarbonate are reference examples. A tendency of forming a film having dull white to transparent-like white and a large white ratio was observed.

Formulations 17 and 18 subjected to an experiment are formulations using the film base and a salt consisting of a mixture of sodium polyphosphate and sodium pyrophosphate, and the above film base and a salt consisting of a mixture of sodium polyphosphate and disodium hydrogen phosphate, respectively. In these cases, films having a large white ratio and transparent-like whiteness were formed.

### Examples

Now, the present invention will be more specifically described referring to Examples described below; however, the present invention is not limited to these.

**Example 1** To an about 34% gelatin solution, an aqueous solution containing 5 parts by weight of sodium polyphosphate relative to 100 parts by weight of the gelatin was added to prepare a film-forming aqueous solution. This solution was poured on a glass plate applied with a release agent and heated to about 60°C and dried at room temperature to form a white film.

### Example 2: Colorability of film

To an about 34% gelatin solution, an aqueous solution containing 5 parts by weight of sodium polyphosphate and 1 part by weight of gardenia red colorant relative to 100 parts by weight of the gelatin was added to prepare an aqueous film-forming solution. This solution was poured on a glass plate applied with a release agent and heated to about 60°C and dried at room temperature to obtain a colored film.

To an about 34% gelatin solution, an aqueous solution containing 5 parts by weight of sodium polyphosphate and 1 part by weight of gardenia blue colorant relative to 100 parts by weight of the gelatin was added to prepare an aqueous film-forming solution. This solution was poured on a glass plate applied with a release agent and heated to about 60°C and dried at room temperature to obtain a colored film. In both cases, it was confirmed that an opaque colored film is prepared.

All salts above were used in an amount of 2.5 parts by weight relative to 100 parts by weight of the film base.

### Example 3: Ink liquid composed of film-forming composition containing an opacifying agent

An ink liquid containing gelatin as the film-forming polymer and an opacifying agent, as shown in the following Table 2 was prepared.

**Table 2: Formulation of ink liquid**

| Formulation | |
|---|---|
| Gelatin | 100 |
| Sodium polyphosphate | 7.5 |
| White ratio | ⊚ |
| Degree of whiteness | ⊚ |
| Peeling | None |

The white ratio and degree of whiteness were visually evaluated, and the criteria were as follows.

The "white ratio" represents the area percentage of whitened parts regardless of the "degree of whiteness" and the "degree of whiteness" refers to the density of a white-looking portion.
White ratio: ⊚ 100 to 90%
Degree of whiteness: ⊚ pure white

To an about 34% gelatin solution, an aqueous solution containing 7.5 parts by weight of sodium polyphosphate relative to 100 parts by weight of the gelatin was added to prepare a film-forming aqueous solution. Using a template having pattern cuts, patterns were printed and heat-dried at 60°C for 15 minutes. As a result, as shown in Figure 1, printing excellent in white ratio and degree of whiteness emerged. When a peeling test was carried out by cellophane tape, no peeling occurred.

From the above experiments, it was confirmed that gelatin used as the film-forming polymer in the above formulations can be used for a solution for hard capsules and also applied to ink. Owing to use of an opacifying agent according to the present invention and an ordinary coloring agent in ink or a film-forming polymer for painting, white and colored opaque printed matters, drawings, painted products in accordance with the objects can be obtained.

### [Industrial Applicability]

The film-forming composition containing a polymer component of gelatin or pullulan dissolved in water and an opacifying agent consisting of a salt can provide a whitened or opacified film having light-shielding property without using titanium oxide, whose carcinogenicity has been reported. The film-forming composition is excellent in solubility and can form a white film stable for a long term. If the composition is used together with a pigment or a colorant, an opaque and colored film can be provided. Accordingly, the film-forming composition is extremely useful particularly as a material for capsules to be charged with a content such as a medicine, an animal medicine, an agricultural chemical, a cosmetic and a healthy food, also, a coating material for tablets and grains, an edible film and edible ink.

## Claims

1. A film comprising a film-forming polymer component and an opacifying agent comprising a water-soluble salt, except calcium salt, carbonate and bicarbonate,
wherein the film-forming polymer component comprises one selected from the group consisting of gelatin and pullulan.

2. A film according to claim 1 wherein the opacifying agent comprises one or more water-soluble salts.

3. The film according to claim 1 or 2, wherein the film-forming polymer component comprises gelatin.

4. The film according to any one of the preceding claims, wherein the salt is a sodium salt, a potassium salt, an ammonium salt or a magnesium salt.

5. The film according to any one of the preceding claims wherein the salt is selected from the group consisting of sodium malate, sodium citrate, disodium hydrogen phosphate, sodium dihydrogen phosphate, sodium succinate, sodium polyphosphate, sodium pyrophosphate, dipotassium hydrogen phosphate, potassium dihydrogen phosphate, potassium acetate, ammonium chloride, magnesium chloride and magnesium sulfate.

6. The film according to claim 3, wherein one or more water-soluble salt are selected from phosphates including hydrogen phosphate, dihydrogen phosphate, pyrophosphate and polyphosphate.

7. The film according to any one of the preceding claims, wherein a degree of opacity is adjusted with a total amount of the salt being in the range of 0.5 to 30 parts by weight with respect to a total amount of 100 parts by weight of the film-forming polymer component in terms of dry weight.

8. The film according to claim 7, wherein a degree of opacity is adjusted with a total amount of the salt being in the range of 1 to 15 parts by weight with respect to a total amount of 100 parts by weight of the film-forming polymer component in terms of dry weight.

9. The film according to claim 7, wherein a degree of opacity is adjusted with a total amount of the salt being in the range of 2 to 15 parts by weight with respect to a total amount of 100 parts by weight of the film-forming polymer component in terms of dry weight.

10. The film according to any one of the preceding claims, colored in a color other than white by adding a dye or a pigment.

11. A capsule suitable for oral delivery which is formed of the film according to any one of the preceding claims.

12. The capsule according to claim 11, wherein the capsule is free from a white pigment.

13. The capsule according to claim 11 or 12, wherein the combination of the film forming polymer component and the salt is,
gelatin and sodium polyphosphate,
gelatin and sodium pyrophosphate,
gelatin and sodium citrate,
gelatin and disodium hydrogen phosphate,
gelatin and sodium dihydrogen phosphate,
gelatin and dipotassium hydrogen phosphate,
gelatin and potassium dihydrogen phosphate,
gelatin, sodium polyphosphate and sodium pyrophosphate, or
gelatin, sodium polyphosphate and disodium hydrogen phosphate.

14. The capsule according to according to claim 11 or 12, wherein the combination of the film forming polymer component and the salt is,
gelatin and sodium polyphosphate,
gelatin and sodium pyrophosphate,
gelatin and disodium hydrogen phosphate,
gelatin and sodium dihydrogen phosphate,
gelatin and dipotassium hydrogen phosphate,
gelatin and potassium dihydrogen phosphate,
gelatin, sodium polyphosphate and sodium pyrophosphate, or
gelatin, sodium polyphosphate and disodium hydrogen phosphate,

15. A film-forming composition to form a film or a capsule according to any one of claims 11 to 14, comprising a film-forming polymer component dissolved in water and an opacifying agent comprising a salt.

16. Use of a salt as an opacifying agent in the manufacture of films or capsules according to any one of claims 1 to 14, **characterized in that** said salt is selected from the group consisting of sodium salts, potassium salts, ammonium salts and magnesium salt.

## Patentansprüche

1. Film, der eine filmbildende Polymerkomponente und ein Trübungsmittel umfasst, das eine wasserlösliches Salz außer Calciumsalz, Carbonat und Bicarbonat umfasst, wobei die filmbildende Polymerkomponente eine umfasst, die aus der Gruppe ausgewählt ist, die aus Gelatine und Pullulan besteht.

2. Film nach Anspruch 1, wobei das Trübungsmittel ein oder mehrere wasserlösliche Salze umfasst.

3. Film nach Anspruch 1 oder 2, wobei die filmbildende Polymerkomponente Gelatine umfasst.

4. Film nach einem der vorhergehenden Ansprüche, wobei das Salz ein Natriumsalz ein Kaliumsalz, ein Ammoniumsalz oder ein Magnesiumsalz ist.

5. Film nach einem der vorhergehenden Ansprüche, wobei das Salz aus der Gruppe ausgewählt ist, bestehend aus Natriummalat, Natriumcitrat, Dinatriumhydrogenphosphat, Natriumdihydrogenphosphat, Natriumsuccinat, Natriumpolyphosphat, Natriumpyrophosphat, Dikaliumhydrogenphosphat, Kaliumdihydrogenphosphat, Kaliumacetat, Ammoniumchlorid, Magnesiumchlorid und Magnesiumsulfat.

6. Film nach Anspruch 3, wobei ein oder mehrere wasserlösliche Salze aus Phosphaten ausgewählt sind, die Hydrogenphosphat, Dihydrogenphosphat, Pyrophosphat und Polyphosphat beinhalten.

7. Film nach einem der vorhergehenden Ansprüche, wobei ein Trübungsgrad mit einer Gesamtmenge des Salzes im Bereich von 0,5 bis 30 Gewichtsanteilen bezogen auf eine Gesamtmenge von 100 Gewichtsanteilen der filmbildenden Polymerkomponente hinsichtlich des Trockengewichts eingestellt wird.

8. Film nach Anspruch 7, wobei ein Trübungsgrad mit einer Gesamtmenge des Salzes im Bereich von 1 bis 15 Gewichtsanteilen bezogen auf eine Gesamtmenge von 100 Gewichtsanteilen der filmbildenden Polymerkomponente hinsichtlich des Trockengewichts eingestellt wird.

9. Film nach Anspruch 7, wobei ein Trübungsgrad mit einer Gesamtmenge des Salzes im Bereich von 2 bis 15 Gewichtsanteilen bezogen auf eine Gesamtmenge von 100 Gewichtsanteilen der filmbildenden Polymerkomponente hinsichtlich des Trockengewichts eingestellt wird.

10. Film nach einem der vorhergehenden Ansprüche, der durch Hinzufügen eines Farbstoffs oder eines Pigments in einer anderen Farbe als weiß eingefärbt wird.

11. Kapsel, die für eine orale Verabreichung geeignet ist, die aus dem Film nach einem der vorhergehenden Ansprüche gebildet ist.

12. Kapsel nach Anspruch 11, wobei die Kapsel frei von einem weißen Pigment ist.

13. Kapsel nach Anspruch 11 oder 12, wobei die Kombination aus der filmbildenden Polymerkomponente und dem Salz
Gelatine und Natriumpolyphosphat,
Gelatine und Natriumpyrophosphat,
Gelatine und Natriumcitrat,
Gelatine und Dinatriumhydrogenphosphat,
Gelatine und Natriumdihydrogenphosphat,
Gelatine und Dikaliumhydrogenphosphat,
Gelatine und Kaliumdihydrogenphosphat,
Gelatine, Natriumpolyphosphat und Natriumpyrophosphat oder
Gelatine, Natriumpolyphosphat und Dinatriumhydrogenphosphat ist.

14. Kapsel nach Anspruch 11 oder 12, wobei die Kombination aus der filmbildenden Polymerkomponente und dem Salz
Gelatine und Natriumpolyphosphat,
Gelatine und Natriumpyrophosphat,
Gelatine und Dinatriumhydrogenphosphat,
Gelatine und Natriumdihydrogenphosphat,
Gelatine und Dikaliumhydrogenphosphat,
Gelatine und Kaliumdihydrogenphosphat,
Gelatine, Natriumpolyphosphat und Natriumpyrophosphat oder
Gelatine, Natriumpolyphosphat und Dinatriumhydrogenphosphat ist.

15. Filmbildende Zusammensetzung, um einen Film oder eine Kapsel nach einem der Ansprüche 11 bis 14 zu bilden, die eine filmbildende Polymerkomponente, die in Wasser gelöst ist, und ein Trübungsmittel umfasst, das ein Salz umfasst.

16. Verwendung eines Salzes als ein Trübungsmittel bei der Herstellung von Filmen oder Kapseln nach einem der Ansprüche 1 bis 14, **dadurch gekennzeichnet, dass** Salz aus der Gruppe ausgewählt ist, bestehend aus Natriumsalzen, Kaliumsalzen, Ammoniumsalzen und Magnesiumsalzen.

## Revendications

1. Film comprenant un composant polymère de formation de film et un agent opacifiant comprenant un sel soluble dans l'eau, à l'exception du sel de calcium, du carbonate et du bicarbonate,
dans lequel le composant polymère de formation de film comprend un composant choisi dans le groupe constitué de la gélatine et du pullulane.

2. Film selon la revendication 1, dans lequel l'agent opacifiant comprend un ou plusieurs sels solubles dans l'eau.

3. Film selon la revendication 1 ou 2, dans lequel le composant polymère de formation de film comprend de la gélatine.

4. Film selon l'une quelconque des revendications précédentes, dans lequel le sel est un sel de sodium, un sel de potassium, un sel d'ammonium ou un sel de magnésium.

5. Film selon l'une quelconque des revendications précédentes, dans lequel le sel est choisi dans le groupe constitué par le malate de sodium, le citrate de sodium, l'hydrogénophosphate disodique, le dihydrogénophosphate de sodium, le succinate de sodium, le polyphosphate de sodium, le pyrophosphate de sodium, l'hydrogénophosphate dipotassique, le dihydrogénophosphate de potassium, l'acétate de potassium, chlorure d'ammonium, le chlorure de magnésium et le sulfate de magnésium.

6. Film selon la revendication 3, dans lequel un ou plusieurs sels solubles dans l'eau sont choisis parmi les phosphates y compris l'hydrogénophosphate, le dihydrogénophosphate, le pyrophosphate et le polyphosphate.

7. Film selon l'une quelconque des revendications précédentes, dans lequel un degré d'opacité est ajusté avec une quantité totale de sel se situant dans la plage de 0,5 à 30 parties en poids par rapport à une quantité totale de 100 parties en poids du composant polymère de formation de film en termes de poids à sec.

8. Film selon la revendication 7, dans lequel un degré d'opacité est ajusté avec une quantité totale du sel se situant dans la plage de 1 à 15 parties en poids par rapport à une quantité totale de 100 parties en poids du composant polymère de formation de film en poids à sec.

9. Film selon la revendication 7, dans lequel un degré d'opacité est ajusté avec une quantité totale du sel se situant dans la plage de 2 à 15 parties en poids par rapport à une quantité totale de 100 parties en poids du composant polymère de formation de film en poids à sec.

10. Film selon l'une quelconque des revendications précédentes, coloré dans une couleur autre que le blanc par addition d'un colorant ou d'un pigment.

11. Capsule convenant à l'administration orale qui est formée du film selon l'une quelconque des revendications précédentes.

12. Capsule selon la revendication 11, dans laquelle la capsule est exempte de pigment blanc.

13. Capsule selon la revendication 11 ou 12, dans laquelle la combinaison du composant polymère de formation de film et du sel est,
la gélatine et le polyphosphate de sodium,
la gélatine et le pyrophosphate de sodium,
la gélatine et le citrate de sodium,
la gélatine et l'hydrogénophosphate disodique,
la gélatine et le dihydrogénophosphate de sodium,
la gélatine et l'hydrogénophosphate dipotassique,
la gélatine et le dihydrogénophosphate de potassium,
la gélatine, polyphosphate de sodium et le pyrophosphate de sodium, ou
la gélatine, le polyphosphate de sodium et l'hydrogénophosphate disodique.

14. Capsule selon la revendication 11 ou 12, dans laquelle la combinaison du composant polymère de formation de film et du sel est,
la gélatine et le polyphosphate de sodium,
la gélatine et le pyrophosphate de sodium,
la gélatine et le phosphate disodique,
la gélatine et le phosphate monosodique,
la gélatine et le phosphate dipotassique,
la gélatine et le phosphate monopotassique,
la gélatine, le polyphosphate de sodium et le pyrophosphate de sodium, ou
la gélatine, le polyphosphate de sodium et l'hydrogénophosphate disodique.

15. Composition de formation de film pour former un film ou une capsule selon l'une quelconque des revendications 11 à 14, comprenant un composant polymère de formation de film dissous dans l'eau et un agent opacifiant comprenant un sel.

16. Utilisation d'un sel en tant qu'agent opacifiant dans la fabrication de films ou capsules selon l'une quelconque des revendications 1 à 14, **caractérisée en ce que** ledit sel est choisi dans le groupe constitué par les sels de sodium, les sels de potassium, les sels d'ammonium et le sel de magnésium.
